# DEMANDE DE BREVET EUROPEEN

(11) **EP 4 604 090 A1**
(43) Date de publication de la demande: **20.08.2025**
(21) Numéro de dépôt: 25157216.0
(22) Date de dépôt: 11.02.2025
(51) Int. Cl.: G08B 21/06, A61B 5/18

(54) **SYSTÈME TRANSPORTABLE D'ÉVALUATION D'UN NIVEAU DE FATIGUE ET PROCÉDÉ D' ÉVALUATION ASSOCIÉ**

(30) Priorité: 13.02.2024 FR 2401396
(71) Demandeur: THALES, 92190 Meudon (FR)
(72) Inventeur: BECOUARN, Loïc, 33700 MERIGNAC (FR); BERTHELOT, Bastien, 33700 MERIGNAC (FR); IBANEZ, Vincent, 33700 MERIGNAC (FR); HOURLIER, Sylvain, 33700 MERIGNAC (FR); GRANIER, Lionel, 33700 MERIGNAC (FR)
(74) Mandataire: Lavoix

(57) **Abrégé**

La présente invention concerne un système (10) transportable d'évaluation d'un niveau de fatigue d'au moins un opérateur.

Le système comprend : un boitier (12), une caméra (14) configurée pour acquérir des images d'un visage de l'opérateur, un capteur (16) configuré pour mesurer un rythme cardiaque de l'opérateur, un calculateur (18) et un dispositif d'interaction homme-machine (40).

Le calculateur est configuré pour recevoir les images acquises depuis la caméra et la mesure du rythme cardiaque depuis le capteur, et configuré pour traiter les images et la mesure pour calculer un niveau de fatigue de l'opérateur.

Le dispositif d'interaction homme-machine est configuré pour émettre, une alerte si le niveau de fatigue calculé est supérieur à un seuil prédéfini.

Le calculateur et le dispositif d'interaction homme-machine sont au moins partiellement intégrés dans le boiter. La caméra et le capteur sont intégrés ou propres à être rangés dans le boitier.

## Description

La présente invention concerne un système transportable d'évaluation objective d'un niveau de fatigue d'au moins un opérateur.

La présente invention concerne le domaine de l'évaluation objective du niveau de fatigue en particulier d'opérateurs dans un contexte professionnel.

Dans certaines professions, le niveau de fatigue des opérateurs est un enjeu fondamental, notamment lorsque la profession de l'opérateur consiste à prendre des décisions pouvant mettre sa vie ou la vie d'autres personnes en péril.

Il est connu qu'un niveau de fatigue avancé altère les performances cérébrales, notamment le temps de réaction et la qualité des décisions prises.

Ainsi, lorsqu'un opérateur est fatigué, la qualité des décisions prises est altérée.

En outre, dans un contexte de fatigue avancée, l'opérateur est susceptible de s'assoupir ou même de s'endormir, le rendant ainsi inapte aux tâches qui lui incombent.

En particulier, dans le domaine aéronautique, le personnel navigant, et en particulier le ou les pilotes, ont pour tâche de garantir la sécurité des passagers et leur transport jusqu'à leur point de destination.

Dans les compagnies aériennes, des systèmes de gestion du risque lié à la fatigue, aussi appelés système FRMS (de l'anglais, *Fatigue Risk Management System*) ont été mis en place. Ces systèmes se fondent sur des questionnaires déclaratifs de niveau de fatigue associés à un modèle biomathématique de prévision d'une évolution de la fatigue au cours du vol. Ces systèmes ont pour objectif de prévoir des phases de somnolences durant le vol.

Toutefois, ces systèmes sont hautement subjectifs puisque limités par les déclarations de l'opérateur avant le vol. Ainsi, il existe un fort biais associé à l'état dans lequel l'opérateur se sent lors de sa déclaration. Cet état est par exemple fortement influencé par sa consommation alimentaire, son niveau de stress ou son excitation au moment où il effectue sa déclaration.

En outre, il n'est pas judicieux de demander à l'opérateur de refaire une déclaration lors de son travail puisque l'interruption risquerait de le déconcentrer et serait susceptible d'induire des risques pour lui ou pour les passagers.

Il existe donc un besoin pour un système d'évaluation non-interruptive et plus objective de la fatigue supprimant le biais déclaratif et permettant de mesurer la fatigue d'un opérateur sans le détourner de son travail.

A cet effet, la présente invention concerne un système transportable d'évaluation d'un niveau de fatigue d'au moins un opérateur, le système comprenant :
- un boitier ;
- une caméra configurée pour acquérir des images d'un visage de l'opérateur ;
- un capteur configuré pour mesurer un rythme cardiaque de l'opérateur ;
- un calculateur configuré pour :
   ∘ recevoir, depuis la caméra, les images acquises du visage de l'opérateur,
   ∘ recevoir, depuis le capteur, une mesure du rythme cardiaque de l'opérateur, et
   ∘ traiter les images et la mesure reçues pour calculer un niveau de fatigue de l'opérateur ; et
- un dispositif d'interaction homme-machine configuré pour émettre, à destination de l'opérateur et/ou d'un administrateur, une alerte si le niveau de fatigue calculé est supérieur à un seuil prédéfini ;
le calculateur et le dispositif d'interaction homme-machine étant au moins partiellement intégrés dans le boiter.
la caméra et le capteur étant intégrés ou propres à être rangés dans le boitier.

Le capteur de rythme cardiaque et la caméra permettent d'acquérir des données représentatives du niveau de fatigue de l'opérateur sans que l'opérateur n'ait besoin de se déconcentrer de sa tâche en cours.

En outre, le fait que le capteur, la caméra, le calculateur et le dispositif d'interaction homme-machine soit intégrés ou propres à être intégré dans le boitier rend le système facilement transportable et permet donc une évaluation du niveau de fatigue : avant, pendant et/ou après, une mission de l'opérateur.

Selon des modes de réalisation particuliers de l'invention, le système comprend une ou plusieurs des caractéristiques suivantes, prises isolément ou suivant toutes les combinaisons techniquement possibles :
- le capteur configuré pour mesurer le rythme cardiaque est un bracelet configuré pour être positionné autour d'un poignet de l'opérateur pour mesurer son rythme cardiaque ;
- la caméra est propre à acquérir des images du visage au moins d'un opérateur qui est localisé à une distance comprise entre 30 et 130cm du boitier, et selon un débattement angulaire défini autour d'un axe optique de la caméra, au moins égal 30° ;
- le boitier comprend une première et une deuxième coques déplaçables entre une position ouverte et une position fermée,
   la première coque définissant un premier compartiment propre à stocker la caméra et une zone de réception du capteur,
   le capteur étant amovible de la zone de réception ;
- la première coque définit une surface destinée à être au moins partiellement en contact contre la deuxième coque lorsque les première et deuxième coques sont dans la position fermée,
   la caméra définissant un axe optique dont un angle d'élévation par rapport à la dite surface est compris entre 30 et 40 degrés, et dont un angle de positionnement autour d'un axe perpendiculaire à ladite surface est compris entre 55 et 65 degrés ;
- le boitier intègre en outre une alimentation configurée pour alimenter en énergie électrique la caméra, le capteur de rythme cardiaque, le calculateur et le dispositif d'interaction homme-machine ;
- le calculateur est configuré pour déterminer des marqueurs faciaux de l'opérateur à partir des images acquises et pour calculer le niveau de fatigue de l'opérateur en outre à partir des marqueurs faciaux déterminés ;
- le système comprend plusieurs capteurs de rythme cardiaque, chacun étant propre à mesurer le rythme cardiaque d'un opérateur respectif,
   le système comprenant en outre l'un parmi :
      ∘ plusieurs caméras chacune configurée pour acquérir des images d'un visage d'un opérateur respectif, et
      ∘ une unique caméra configurée pour acquérir des images d'un visage de chacun de plusieurs opérateurs,
   le calculateur étant configuré pour calculer un niveau de fatigue de chaque opérateur à partir de la mesure du rythme cardiaque dudit opérateur et des images acquises du visage dudit opérateur ; et
- le dispositif d'interaction homme-machine comprend un écran d'affichage et/ou un haut-parleur, et optionnellement un vibreur intégré dans le capteur,
   le dispositif d'interaction homme-machine étant en outre configuré pour indiquer à l'opérateur lorsque l'acquisition d'images et la mesure du rythme cardiaque sont terminées,
   de préférence, le dispositif d'interaction homme-machine étant configuré en outre pour acquérir des données relatives à l'opérateur ou à chaque opérateur, de préférence encore, le dispositif d'interaction homme-machine étant configuré pour signaler à l'opérateur s'il sort du champ de vision de la caméra et/ou si les yeux de l'opérateur sortent du champ de vision de la caméra.

L'invention a également pour objet un procédé d'évaluation d'un niveau de fatigue d'un opérateur, le procédé étant mis en oeuvre par un tel système transportable et comprenant les étapes suivantes :
- acquisition de plusieurs images du visage de l'opérateur,
- mesure du rythme cardiaque de l'opérateur pour obtenir une mesure du rythme cardiaque,
- traitement des images acquises et de la mesure du rythme cardiaque pour calculer le niveau de fatigue de l'opérateur, et
- si le niveau de fatigue est supérieur à un seuil prédéfini, émission d'une alerte à destination de l'opérateur et/ou d'un administrateur.

Le système selon l'invention ainsi que ses modes réalisations seront mieux compris à la lecture de la description qui suit, donnée à titre d'exemple non-limitatif et faite en référence aux dessins annexés sur lesquels :
- la figure 1 est une représentation schématique d'un système transportable d'évaluation d'un niveau de fatigue selon un premier mode de réalisation de l'invention ;
- la figure 2 est une représentation schématique en détail de la caméra du système de la figure 1 ;
- la figure 3 est une représentation schématique en coupe du système de la figure 1 ;
- la figure 4 est un organigramme d'un procédé d'évaluation d'un niveau de fatigue ; et
- la figure 5 est une représentation schématique d'un système transportable d'évaluation d'un niveau de fatigue selon un deuxième mode de réalisation de l'invention.

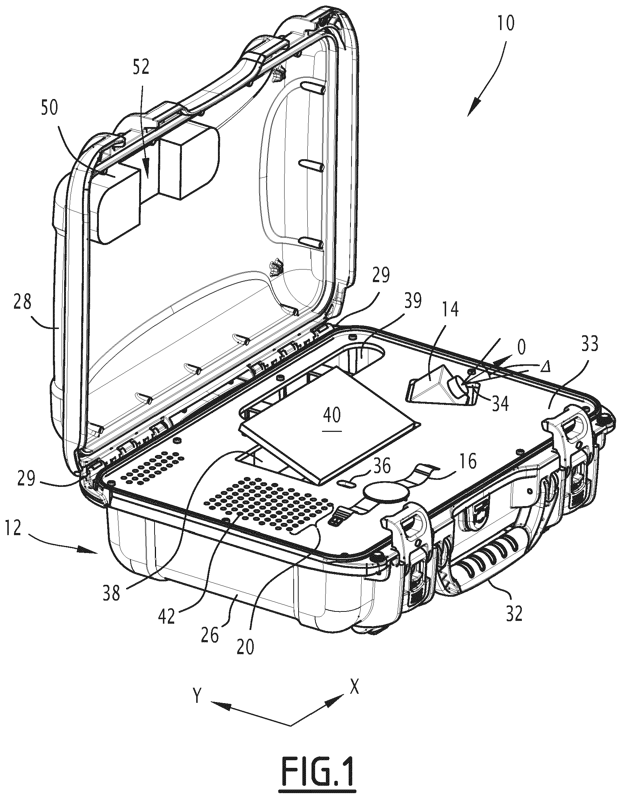

En référence à la figure 1, on décrit un système transportable 10 d'évaluation d'un niveau de fatigue d'au moins un opérateur.

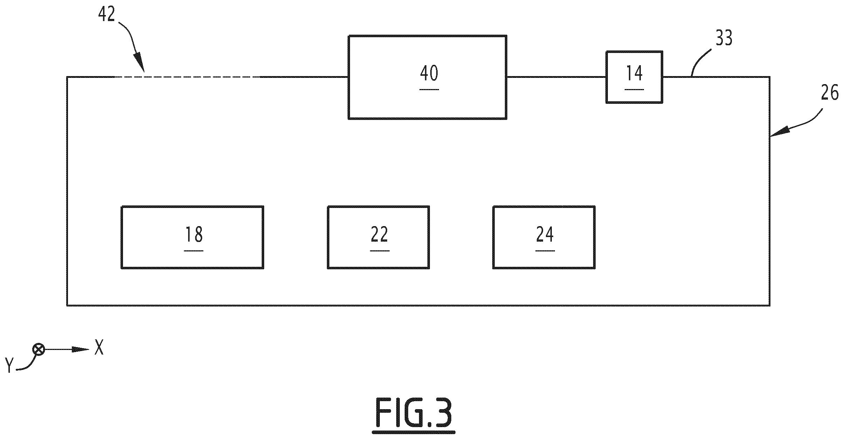

Le système 10 comprend un boitier 12, une caméra 14, un capteur 16, un calculateur 18, un dispositif d'interaction homme-machine 40 et optionnellement une alimentation 22 représentée sur la figure 3 et une antenne réseau 24, visibles sur la figure 3.

Le calculateur 18, le dispositif d'interaction homme-machine 40 et optionnellement l'alimentation et l'antenne réseau 24 sont intégrés dans le boiter 12. La caméra 14 et le capteur 16 sont intégrés ou propres à être rangés dans le boitier 12.

Le boitier 12 est destiné à être posé sur une surface, dite surface de support, telle qu'une table ou un tableau de bord d'un cockpit.

Le boitier 12 comprend préférentiellement une première coque 26 et une deuxième coque 28 déplaçables entre une position ouverte et une position fermée.

Préférentiellement, le boitier 12 comprend des moyens 29 formant pivot rendant solidaire les première 26 et deuxième 28 coque à un de leurs bords respectifs et permettant les rendre déplaçable l'un par rapport à l'autre.

Préférentiellement également, le boitier 12 comprend en outre des moyens de verrouillage de la première 26 et de la deuxième 28 coques dans la position fermée.

En outre, le boitier 12 comprend avantageusement sur une de ses coques 26, 28, une poignée 32 pour transporter le boitier lorsque les coques 26, 28 sont en position fermée.

Les moyens de verrouillage sont préférentiellement positionnés de part et d'autre de la poignée 32 sur le même bord.

Les dimensions du boitier 12 sont par exemple telles qu'il respecte les contraintes dimensionnelles d'un bagage cabine. Par exemple le boitier 12 présente des dimensions inférieures à 55 x 35 x 25 centimètres. Avantageusement, le boitier 12 se présente sous la forme d'une valise.

La première coque 26 a une forme sensiblement parallélépipédique définissant une surface non-représentée propre à être en contact avec la surface de support, et une surface 33, dite surface opposée 33, opposée à cette surface non-représentée. La surface opposée 33 est sensiblement rectangulaire et définit une première direction d'extension X et une deuxième direction d'extension Y perpendiculaires entre elles.

La surface opposée 33 est destinée à être au moins partiellement en contact avec la deuxième coque 28 lorsque les première 26 et deuxième 28 coques sont dans la position fermée.

La première direction d'extension X est sensiblement parallèle au bord de la première coque 26 présentant les moyens formant pivot 29 avec la deuxième coque 28, la deuxième direction d'extension Y étant sensiblement perpendiculaire audit bord.

La première coque 26 définit préférentiellement un premier compartiment 34 propre à stocker la caméra 14.

Optionnellement, la première coque 26 est propre à recevoir le capteur 16 sur la surface opposée 33. La première coque 26 définit alors préférentiellement une zone de réception comprenant un orifice 36 pour le passage d'un câble de chargement, aussi appelé chargeur, du capteur 16 lorsqu'il repose sur la surface opposée 33, comme représenté sur la figure 1.

Préférentiellement, la première coque 26 définit en outre un deuxième compartiment 38 propre à stocker au moins partiellement le dispositif d'interaction homme-machine 40, et optionnellement un troisième compartiment 39 propre à stocker au moins partiellement l'alimentation 22, préférentiellement un câble de l'alimentation non représenté.

Selon un exemple non-représenté, le dispositif d'interaction homme-machine 40 et le calculateur 18 sont faits d'un même élément. Autrement dit, un seul bloc physique comprend le dispositif d'interaction homme-machine 40 et le calculateur 18

Avantageusement, la première coque 26 définit en outre une aération 20 formée par des trous de ventilation 42 traversant la surface opposée 33 de part en part, pour assurer une ventilation d'éléments intégré dans coque, sous ladite surface 33. Ces éléments seront décrits ci-après.

Comme il sera décrit ci-après, le capteur 16 est préférentiellement amovible de la coque 26. Dans le mode de réalisation représenté sur la figure 1, la caméra 14 et le dispositif d'interaction homme-machine 40 ne sont respectivement pas amovibles du premier compartiment 34 et du deuxième compartiment 38.

Les premier 34, deuxième 38, et troisième 39 compartiments ainsi que l'orifice 36 sont par exemple ménagés dans la surface opposée 33.

La caméra 14 est par exemple configurée pour acquérir des images d'un visage de l'opérateur positionné proche du boitier 12.

Dans le mode de réalisation de la figure 1, la caméra 14 est fixe par rapport au boitier 12, i.e. intégrée dans le premier compartiment 34 et non-amovible de ce compartiment 34.

La caméra 14 définit un axe optique O.

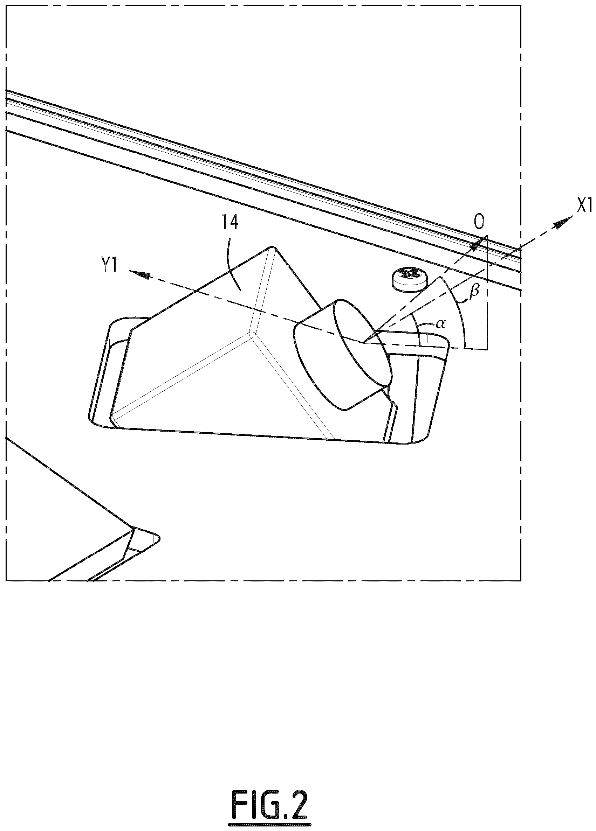

Comme représenté sur la figure 2 illustrant la caméra 14 en détail, on définit un repère centré sur la caméra 14 et défini par un premier axe X1 s'étendant selon la première direction X et par un deuxième axe Y1 s'étendant selon la deuxième direction Y. Dans ce repère, l'axe optique O forme un angle de positionnement *α* avec le premier axe X1. L'angle de positionnement *α* est préférentiellement compris entre 55 et 65 degrés.

On comprend que l'angle de positionnement *α* est une angle autour d'un axe non-représenté perpendiculaire à la surface opposée 33.

L'axe optique O forme un angle d'élévation *β* par rapport à la surface opposé 33, compris entre 30 et 40 degrés.

Sur la figure 2, le repère X1, Y1 est représenté en tiret et les projections de l'axe optique O sur ce repère sont représentées en trait mixte.

Les valeurs de l'angle de positionnement *α*, de l'angle d'élévation *β* et du débattement angulaire Δ permettent à la caméra 14 d'acquérir des images du visage de l'opérateur même si l'opérateur n'est pas positionné face au boitier 12. Ainsi, l'opérateur est libre d'effectuer d'autres tâches pendant que la caméra 14 acquiert des images de son visage.

En outre, et de nouveau en référence à la figure 1, la caméra 14 est propre à acquérir des images du visage d'un opérateur qui est localisé à une distance comprise entre 30cm et 130cm du boitier 12, et selon un débattement angulaire Δ, défini autour de l'axe optique O, au moins égale à 30°. Préférentiellement, le débattement angulaire est égal à plus ou moins 20 degrés selon un plan parallèle à la surface opposée 33, i.e. en horizontal, et plus ou moins 15 degrés selon un plan perpendiculaire à la surface opposée 33, i.e. en vertical.

Optionnellement, la caméra 14 est une caméra infrarouge.

Selon un exemple non-représenté, des illuminateurs, par exemple des LEDs infrarouges, sont agencées proche de la caméra 14 pour éclairer l'opérateur lors de l'acquisition d'images par la caméra 14. Ceci permet que les images du visage de l'opérateur soient exploitables même en environnement sombre.

Le capteur 16 est configuré pour mesurer un rythme cardiaque de l'opérateur.

Par exemple et comme illustré sur la figure 1, le capteur 16 est un bracelet configuré pour être positionné autour d'un poignet de l'opérateur pour mesurer son rythme cardiaque.

Le capteur 16 est propre à reposer sur la surface opposée 33. Par exemple, le capteur 16 présente un cadran comprenant la circuiterie électronique destinée à effectuer des mesures.

Pour maintenir le capteur 16 en place lorsqu'il repose sur la surface opposée 33 en position fermée, la deuxième coque 28 comprend préférentiellement une portion extrudée 50 en saillie de la deuxième coque 28, et destinée à être contact avec le capteur 16 en position fermée. Préférentiellement, lorsque le capteur 16 présente un cadrant, la portion extrudée 50 définit un retrait 52 dont une dimension correspond à un diamètre du cadran pour coopérer avec le capteur 16 en position fermée.

La portion extrudée 50 est par exemple une mousse.

On comprend alors que, lorsque le capteur 16 repose sur la surface opposée 33, son bracelet s'étend selon la première direction d'élongation X.

Par exemple, le capteur 16 est configuré pour mesurer le rythme cardiaque de l'opérateur par photopléthysmographie, dit PPG. Cette technique est connue en soit.

Alternativement, le capteur 16 est configuré pour effectuer cette mesure à partir d'une analyse de réponse électrique entre le capteur 16 est le poignet de l'opérateur, ou par analyse de signaux radars se propageant dans le poignet de l'opérateur.

En complément facultatif, le capteur 16 est configuré pour mesure en outre d'autres paramètres physiologique de l'opérateur, tels que la pression artérielle, la saturation en oxygène, la sudation, le taux de déshydratation.

Pour la saturation en oxygène par exemple, de manière connue, le capteur 16 est par exemple configuré pour émettre, en direction de la peau de l'opérateur, et recevoir, un signal lumineux comprenant au moins deux longueurs d'onde. Une première longueur d'onde correspond à une longueur d'onde absorbée par les globules rouges saturés, une deuxième longueur d'onde correspondant à une longueur d'onde absorbée par les globules rouges non-saturés.

Dans cet exemple, le capteur 16 est configuré pour déterminer la saturation en oxygène de l'opérateur par comparaison de l'intensité lumineuse reçue à chacune des deux longueurs d'onde.

Préférentiellement, par l'orifice 36 passe le câble d'un chargeur non-représenté, tel qu'un chargeur à induction ou à contact direct, configuré pour recharger le capteur 16 lorsqu'il est positionné sur la surface opposée 33.

En référence à la figure 3 représentant une vue en coupe de la première coque 26 selon un plan de coupe perpendiculaire à la deuxième direction d'élongation Y, la première coque 26 intègre préférentiellement, sous la surface opposée 33, le calculateur 18, une partie de l'alimentation 22 et l'antenne réseau 24.

Le calculateur 18 est configuré pour recevoir, depuis la caméra 14, les images acquises du visage de l'opérateur. A cet effet, le calculateur 18 est par exemple connecté à la caméra via une liaison filaire non-représentée.

Le calculateur 18 est en outre configuré pour recevoir, depuis le capteur 16, la mesure du rythme cardiaque, par exemple via une connexion sans-fil transitant via l'antenne réseau 24 elle-même connectée au calculateur 18.

Le calculateur 18 est de plus configuré pour traiter les images de la caméra 14 et la mesure, i.e. les données du capteur 16, reçues pour calculer un niveau de fatigue de l'opérateur.

Le niveau de fatigue est par exemple une note donnée sur une échelle de 20 ou de 100.

A cet effet, le calculateur 18 est préférentiellement configuré pour déterminer des marqueurs faciaux de l'opérateur. Les marqueurs faciaux sont des points d'intérêt localisés sur le visage et à partir desquels différentes caractéristiques sont propres à être extraites. A titre d'exemples, lesdites caractéristiques sont : des statistiques de bâillement, d'ouverture des yeux, de direction du regard et de la tête, de fréquence de clignement des yeux, etc.

Le calculateur 18 est préférentiellement configuré pour calculer le niveau de fatigue de l'opérateur en outre à partir des marqueurs faciaux déterminés.

Par exemple, le calculateur 18 est configuré pour extraire les caractéristiques des marqueurs faciaux, par exemple à l'aide de technique telle que celle présentée dans les demandes de brevet FR3133534 et FR3133691.

Le calculateur 18 est ensuite configuré pour fournir ces caractéristiques, ainsi que la mesure, i.e. les données du capteur 16 à un modèle d'intelligence artificielle préalablement entrainé à partir d'un jeu de données labélisé, pour déterminer le niveau de fatigue de l'opérateur.

Par exemple, le modèle d'intelligence artificielle est un réseau de neurones.

Le réseau de neurones comporte une succession ordonnée de couches de neurones dont chacune prend ses entrées sur les sorties de la couche précédente.

Plus précisément, chaque couche comprend des neurones prenant leurs entrées sur les sorties des neurones de la couche précédente, ou bien sur les variables d'entrée pour la première couche.

À chaque neurone est également associée une opération, c'est-à-dire un type de traitement, à effectuer par ledit neurone au sein de la couche de traitement correspondante.

Chaque couche est reliée aux autres couches par une pluralité de synapses. Un poids synaptique est associé à chaque synapse, et chaque synapse forme une liaison entre deux neurones. Chaque poids synaptique est préférentiellement un nombre réel, qui prend des valeurs positives comme négatives. Dans certains cas, chaque poids synaptique est un nombre complexe.

Chaque neurone est propre à effectuer une somme pondérée des valeur(s) reçue(s) de la part des neurones de la couche précédente, chaque valeur étant alors multipliée par le poids synaptique respectif de chaque synapse, ou liaison, entre ledit neurone et les neurones de la couche précédente, puis à appliquer une fonction d'activation, typiquement une fonction non-linéaire, à ladite somme pondérée, et à délivrer en sortie dudit neurone, en particulier aux neurones de la couche suivante qui lui sont connectés, la valeur résultant de l'application de la fonction d'activation. La fonction d'activation permet d'introduire une non-linéarité dans le traitement effectué par chaque neurone. La fonction sigmoïde, la fonction tangente hyperbolique, la fonction de Heaviside, la fonction Unité Linéaire Rectifiée aussi appelée fonction ReLU (de l'anglais, *Rectified Linear Unit*), ou la fonction softmax, sont des exemples de fonction d'activation.

En complément facultatif, chaque neurone est également apte à appliquer, en outre, un facteur multiplicatif, également appelé biais, à la sortie de la fonction d'activation, et la valeur délivrée en sortie dudit neurone est alors le produit de la valeur de biais et de la valeur issue de la fonction d'activation. Le calculateur 18 est ensuite configuré pour communiquer à l'opérateur, le niveau de fatigue via le dispositif d'interaction homme-machine comme il sera décrit ci-après.

En variante, le calculateur 18 est configuré pour transmettre le niveau de fatigue calculé à un dispositif externe au système 10 via l'antenne réseau 24 à destination d'un administrateur.

Comme visible sur la figure 1, le dispositif d'interaction homme-machine 40 comprend par exemple un écran d'affichage, tel qu'un écran tactile, et optionnellement un haut-parleur non-représenté.

Le haut-parleur est par exemple localisé en dessous des trous de ventilation 42.

Alternativement, l'écran d'affichage comprend un écran 40 non-tactile et un clavier non-représenté.

En complément facultatif, le dispositif d'interaction homme-machine 40 est également partiellement intégré au capteur 16, par exemple sous la forme d'un vibreur non-représenté.

Le dispositif d'interaction homme-machine 40 est configuré pour émettre, à destination de l'opérateur et/ou d'un administrateur, une alerte si le niveau de fatigue calculé est supérieur à un seuil prédéfini. 8

A cet effet, si le destinataire de l'alerte est l'opérateur du système 10, l'alerte est par exemple un message affiché sur l'écran tactile 40 ou un signal sonore émis par le haut-parleur.

Si le destinataire de l'alerte est un administrateur, l'alerte est par exemple transmise via l'antenne réseau 24.

En complément facultatif, le dispositif d'interaction homme-machine 40 est configuré pour acquérir des données relatives à l'opérateur ou à chaque opérateur, telle que son sexe, son âge, sa taille, son poids, ou autres.

En outre, le dispositif d'interaction homme-machine 40 est préférentiellement configuré pour indiquer à l'opérateur lorsque l'acquisition d'images et la mesure du rythme cardiaque sont terminées.

A cet effet, préférentiellement, le vibreur intégré au capteur 16 est configuré pour vibrer lorsque l'acquisition d'images et la mesure du rythme cardiaque sont terminées.

Préférentiellement, le dispositif d'interaction homme-machine 40est configuré pour signaler à l'opérateur s'il sort du champ de vision de la caméra 14 lors de l'acquisition d'images, en particulier si ces yeux sortent du champ de vision de la caméra 14. Le champ de vision de caméra est préférentiellement défini par l'angle de positionnement *α*, l'angle d'élévation *β* et le débattement angulaire Δ.

A titre d'exemple, les yeux de l'opérateur peuvent sortir du champ de vision si l'opérateur tourne ou incline la tête ou alors si l'opérateur se déplace et n'est plus en face de la caméra 14.

Avantageusement, pour effectuer un tel signalement à l'opérateur, le vibreur intégré au capteur 16 est configuré pour vibrer.

L'alimentation 22 est propre à fournir de l'énergie électrique à la caméra 14, au capteur de rythme cardiaque 16, au calculateur 18 et au dispositif d'interaction homme-machine 40. Par exemple, l'alimentation 22 comprend un câble propre à être connecté à une fiche de distribution électrique.

En variante, l'alimentation 22 est une batterie.

L'antenne réseau 24 est propre à être connectée à un système réseau local, par exemple via un protocole Wi-Fi^{™} ou Bluetooth^{™}, ou un système réseau global via a protocole cellulaire 4G ou 5G. L'antenne réseau 24 est propre à transférer des données entre le calculateur 18 et un système externe non-représenté, telles que le niveau de fatigue déterminé par le calculateur 18.

Optionnellement, l'antenne réseau 24 est en outre propre à communiquer avec le capteur 16 pour que le calculateur 18 reçoive les mesures du rythme cardiaque.

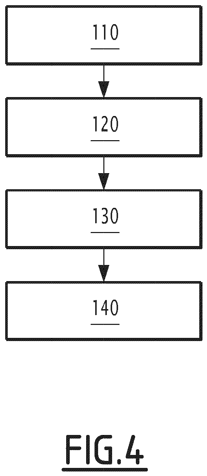

Le fonctionnement du système 10 va maintenant être décrit en référence à la figure 4 illustrant un organigramme d'un procédé d'évaluation d'un niveau de fatigue d'un opérateur. Ce procédé est par exemple mis en oeuvre, avant, pendant ou après un vol.

Initialement, l'opérateur ouvre le boitier 12 et se saisit du capteur 16. Optionnellement, si l'alimentation 22 comprend un câble d'alimentation, l'opérateur connecte le câble à une fiche de distribution électrique.

Si le capteur 16 est un bracelet, l'opérateur le positionne autour de son poignet.

L'opérateur positionne le boitier 12 de sorte à être dans le champ de vision de la caméra 14. On comprend alors que le boitier 12 n'est pas directement en face de l'opérateur mais légèrement décalé sur le côté de sorte à ne pas encombrer la zone en face de l'opérateur pour qu'il puisse effectuer différentes tâches, alors même que son niveau de fatigue est évalué.

Préférentiellement, l'opérateur s'identifie sur le système 10. A cet effet, l'identification de l'opérateur est par exemple réalisée via un code entré manuellement par l'opérateur sur le dispositif d'interaction homme-machine 40, ou par lecture d'un code via NFC ou un QR code lu par la caméra 14.

Le procédé comprend une étape d'acquisition 110, lors de laquelle la caméra 14 acquiert plusieurs images du visage de l'opérateur.

Préférentiellement, lors de l'étape d'acquisition 110, si l'opérateur, et/ou ses yeux, sortent du champ de vision de la caméra, défini par : l'angle de positionnement *α*, l'angle d'élévation *β* et le débattement angulaire Δ, le dispositif d'interaction homme-machine 40 signale que l'opérateur sort du champs de vision de la caméra 14, par exemple en faisant vibrer le vibreur intégré dans le capteur 16, ou alternativement en émettant un son ou en affichant un message.

Le procédé comprend en outre une étape 120 de mesure du rythme cardiaque de l'opérateur pour obtenir une mesure du rythme cardiaque, préférentiellement par le capteur 16.

Préférentiellement les étapes d'acquisition 110 et de mesure 120 sont mises en oeuvre simultanément.

Le procédé comprend en outre une étape 130 de traitement des images acquises et de la mesure du rythme cardiaque pour calculer le niveau de fatigue de l'opérateur.

Comme expliqué précédemment, préférentiellement, le calculateur 18 détermine les marqueurs faciaux de l'opérateur. Puis, le calculateur 18 extrait les caractéristiques des marqueurs faciaux et fournit ces caractéristiques et la mesure, au modèle d'intelligence artificielle préalablement entraîné pour déterminer le niveau de fatigue.

Le procédé comprend ensuite, si le niveau de fatigue est supérieur à un seuil prédéfini, une étape 140 d'émission d'une alerte à destination de l'opérateur et/ou d'un administrateur comme expliqué précédemment.

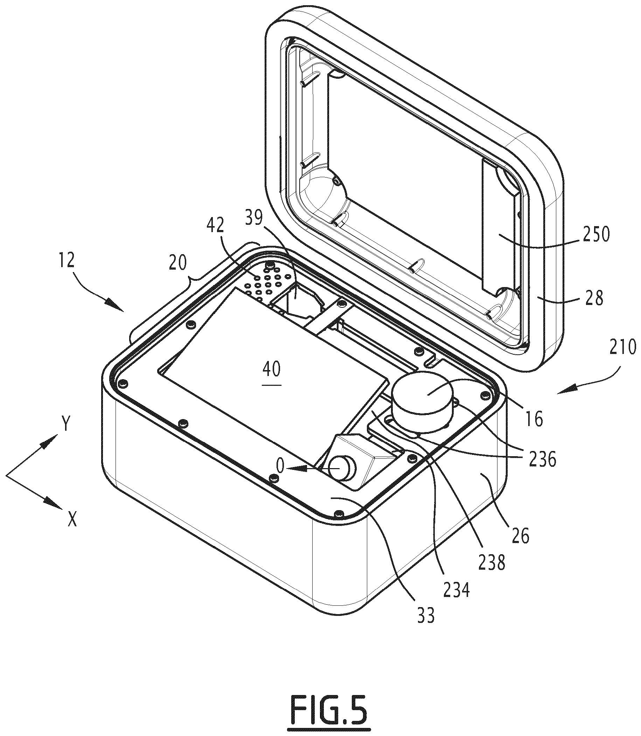

Un second mode de réalisation va maintenant être décrit en référence à la figure 5.

Les éléments communs entre le premier et le deuxième mode de réalisation conservent leur numéro de référence. Seul les éléments distincts portent une référence incrémentée de la valeur 200.

Le deuxième mode de réalisation va être décrit seulement par ses différences avec le premier mode de réalisation de sorte à ce que chaque caractéristique qui n'est pas décrite est identique à la caractéristique correspondante dans le premier mode de réalisation.

Dans ce deuxième mode de réalisation, le système transportable 210 est plus compact que le système transportable 10 du premier mode de réalisation.

Dans le deuxième mode de réalisation, le premier compartiment 234 est contigu du deuxième compartiment 238.

Selon l'exemple illustré sur la figure 5, les premier 234 et deuxième 238 compartiments sont par exemple communs. Autrement dit, selon cette exemple, il n'y a pas de délimitation physique entre le premier 234 et le deuxième 238 compartiments.

Dans ce deuxième mode de réalisation, le premier compartiment 234 s'entend sensiblement selon la première X et la deuxième Y directions.

Dans ce deuxième mode de réalisation, l'axe optique O de la caméra 14 est, en projection sur la surface opposée 33 sensiblement aligné avec la deuxième direction d'élongation Y, dans un sens opposé. Autrement dit, l'angle de positionnement *α* a une valeur sensiblement égale à 90°.

Dans ce deuxième mode de réalisation, la zone de réception du capteur 16 définit préférentiellement trois orifices 236 plutôt qu'un seul dans le premier mode de réalisation. Deux des trois orifices sont alors préférentiellement des fentes, par exemple parallèles entre elles et s'étendant optionnellement selon la première direction d'élongation X. Chacun de ces deux orifices 236 est destiné à recevoir une patte du bracelet du capteur 16 pour son rangement en position fermée. Le troisième orifice 36, non-représenté sur la figure 5, est destiné au passage du câble du chargeur du capteur 16.

Sur la figure 5, uniquement deux des trois orifices 236 sont visibles.

Préférentiellement, la deuxième coque 28 comprend également la portion extrudée 250. Cependant, dans ce deuxième mode de réalisation, la portion extrudée 250 ne présente préférentiellement pas de retrait et n'est en contact avec le capteur 16 que par le cadran de ce dernier, lorsqu'il est présent, en position fermée.

Dans ce deuxième mode de réalisation, les trous de ventilation 42 s'étendent sur une zone plus petite sur la surface opposée 33 que dans le premier mode de réalisation. Le haut-parleur est optionnellement placé sous ces trous de ventilation 42.

Dans ce deuxième mode de réalisation, les moyens de verrouillage sont positionnés sur des bords des première 26 et deuxième 28 coques sensiblement perpendiculaires à celui comportant la poignée 32 et celui comportant les moyens formant pivot. Les moyens formant pivot ne sont pas représentés sur la figure 5.

Ainsi, le système 210 selon le deuxième mode de réalisation est plus compact que le système 10 selon le deuxième mode de réalisation ce qui favorise encore plus l'aspect transportable dudit système 210.

Le fonctionnement du système 210 selon le deuxième mode de réalisation est analogue au fonctionnement du système 10 selon le premier mode de réalisation. Ainsi, le système 210 selon le deuxième mode de réalisation est propre à mettre en oeuvre le procédé d'évaluation du niveau de fatigue décrit précédemment.

Dans le deuxième mode de réalisation, l'axe optique O de la caméra 14 est tel que l'opérateur est placé en face du boitier lors de l'acquisition d'image. Toutefois, la compacité du système 210 selon le deuxième mode de réalisation permet de ne tout de même pas encombrer l'espace en face de l'opérateur.

Alternativement, la caméra 14 est orientée vers le côté droit au gauche du système 10, de sorte que le système puisse être respectivement placé sur la gauche ou la droite de l'opérateur tout en pointant la caméra 14 vers l'opérateur.

Des variantes du système 10, 210 vont maintenant être décrites dans lequel le système est configuré pour évaluer le niveau de fatigue de plusieurs opérateurs simultanément. Ces variantes sont compatibles avec le système 10, 210 selon le premier et le deuxième mode de réalisation.

Dans ces variantes, le système 10, 210 comprend plusieurs capteurs 16. Préférentiellement, le premier boitier 26 comprend alors plusieurs orifices 236, ou triplet d'orifices 236.

Selon une première variante, le système 10, 210 comprend en outre plusieurs caméras 16, chacune configurée pour acquérir des images d'un visage d'un opérateur respectif.

Selon une deuxième variante, le système 10, 210 comprend une unique caméra configurée pour acquérir des images d'un visage de chacun de plusieurs opérateurs.

Dans ces variantes, le calculateur 18 est configuré pour calculer un niveau de fatigue de chaque opérateur à partir de la mesure du rythme cardiaque dudit opérateur et des images acquises du visage dudit opérateur, comme expliqué précédemment pour chaque opérateur.

Selon ces variantes, le système 10, 210 est propre à mettre en oeuvre le procédé d'évaluation du niveau de fatigue décrit précédemment, pour chaque opérateur.

Le système 10, 210 selon l'invention permet d'évaluer la fatigue de l'opérateur sans le déconcentrer de son travail et en s'émancipant du biais déclaratif.

En outre, le système 10, 210 permet une évaluation du niveau de fatigue qui est objective et non-intrusive puisque l'opérateur est complètement passif pendant l'évaluation.

En outre, le fait que le système 10, 210 soit transportable rend son utilisation particulièrement aisée et possible dans de multiples situations.

## Revendications

1. Système (10 ; 210) transportable d'évaluation d'un niveau de fatigue d'au moins un opérateur, le système (10 ; 210) comprenant :
- un boitier (12) ;
- une caméra (14) configurée pour acquérir des images d'un visage de l'opérateur ;
- un capteur (16) configuré pour mesurer un rythme cardiaque de l'opérateur ;
- un calculateur (18) configuré pour :
∘ recevoir, depuis la caméra (14), les images acquises du visage de l'opérateur,
∘ recevoir, depuis le capteur (16), une mesure du rythme cardiaque de l'opérateur, et
∘ traiter les images et la mesure reçues pour calculer un niveau de fatigue de l'opérateur ; et
- un dispositif d'interaction homme-machine (40) configuré pour émettre, à destination de l'opérateur et/ou d'un administrateur, une alerte si le niveau de fatigue calculé est supérieur à un seuil prédéfini ;
le calculateur (18) et le dispositif d'interaction homme-machine (40) étant au moins partiellement intégrés dans le boiter (12) ;
la caméra (14) et le capteur (16) étant intégrés ou propres à être rangés dans le boitier (12).

2. Système (10 ; 210) selon la revendication 1, dans lequel le capteur (16) configuré pour mesurer le rythme cardiaque est un bracelet configuré pour être positionné autour d'un poignet de l'opérateur pour mesurer son rythme cardiaque.

3. Système (10 ; 210) selon la revendication 1 ou 2, dans lequel la caméra (14) est propre à acquérir des images du visage au moins d'un opérateur qui est localisé à une distance comprise entre 30 et 130cm du boitier (12), et selon un débattement angulaire défini autour d'un axe optique (O) de la caméra (14), au moins égal 30°.

4. Système (10 ; 210) selon l'une quelconque des revendications précédentes, dans lequel le boitier (12) comprend une première (26) et une deuxième coques (28) déplaçables entre une position ouverte et une position fermée,
la première coque (26) définissant un premier compartiment (34 ; 234) propre à stocker la caméra (14) et une zone de réception (36 ; 236) du capteur (16),
le capteur (16) étant amovible de la zone de réception.

5. Système (10 ; 210) selon la revendication précédente, dans lequel la première coque (26) définit une surface (33) destinée à être au moins partiellement en contact contre la deuxième coque (28) lorsque les première (26) et deuxième (28) coques sont dans la position fermée,
la caméra (14) définissant un axe optique (O) dont un angle d'élévation (*β*) par rapport à la dite surface (33) est compris entre 30 et 40 degrés, et dont un angle de positionnement (*α*) autour d'un axe perpendiculaire à ladite surface (33) est compris entre 55 et 65 degrés.

6. Système (10 ; 210) selon l'une quelconque des revendications précédentes, dans laquelle le boitier (12) intègre en outre une alimentation (22) configurée pour alimenter en énergie électrique la caméra (14), le capteur de rythme cardiaque (16), le calculateur (18) et le dispositif d'interaction homme-machine (40).

7. Système (10 ; 210) selon l'une quelconque des revendications précédentes, dans lequel le calculateur (18) est configuré pour déterminer des marqueurs faciaux de l'opérateur à partir des images acquises et pour calculer le niveau de fatigue de l'opérateur en outre à partir des marqueurs faciaux déterminés.

8. Système (10 ; 210) selon l'une quelconque des revendications précédentes, dans lequel le système (10 ; 210) comprend plusieurs capteurs de rythme cardiaque (16), chacun étant propre à mesurer le rythme cardiaque d'un opérateur respectif,
le système (10 ; 210) comprenant en outre l'un parmi :
- plusieurs caméras (14) chacune configurée pour acquérir des images d'un visage d'un opérateur respectif, et
- une unique caméra (14) configurée pour acquérir des images d'un visage de chacun de plusieurs opérateurs,
le calculateur (18) étant configuré pour calculer un niveau de fatigue de chaque opérateur à partir de la mesure du rythme cardiaque dudit opérateur et des images acquises du visage dudit opérateur.

9. Système (10 ; 210) selon l'une quelconque des revendications précédentes, dans lequel le dispositif d'interaction homme-machine (40) comprend un écran d'affichage et/ou un haut-parleur, et optionnellement un vibreur intégré dans le capteur (16),
le dispositif d'interaction homme-machine (40) étant en outre configuré pour indiquer à l'opérateur lorsque l'acquisition d'images et la mesure du rythme cardiaque sont terminées, de préférence, le dispositif d'interaction homme-machine (40) étant configuré en outre pour acquérir des données relatives à l'opérateur ou à chaque opérateur,
de préférence encore, le dispositif d'interaction homme-machine (40) étant configuré pour signaler à l'opérateur s'il sort du champ de vision de la caméra (14) et/ou si les yeux de l'opérateur sortent du champ de vision de la caméra (14).

10. Procédé d'évaluation d'un niveau de fatigue d'un opérateur, le procédé étant mis en oeuvre par un système transportable (10 ; 210) selon l'une quelconque des revendications précédentes et comprenant les étapes suivantes :
- acquisition (110) de plusieurs images du visage de l'opérateur,
- mesure (120) du rythme cardiaque de l'opérateur pour obtenir une mesure du rythme cardiaque,
- traitement (130) des images acquises et de la mesure du rythme cardiaque pour calculer le niveau de fatigue de l'opérateur, et
- si le niveau de fatigue est supérieur à un seuil prédéfini, émission (140) d'une alerte à destination de l'opérateur et/ou d'un administrateur.
